# EUROPEAN PATENT APPLICATION

(11) **EP 1 974 742 A1**
(43) Date of publication of application: **01.10.2008**
(21) Application number: 07105215.3
(22) Date of filing: 29.03.2007
(51) Int. Cl.: A61K 39/00, A61K 47/00

(54) **A method for improving the manufacturing process of a tumour vaccine**

(71) Applicant: LipoNova AG, 30625 Hannover (DE)
(72) Inventor: The designation of the inventor has not yet been filed
(74) Representative: von Kreisler Selting Werner

(57) **Abstract**

A method for improving the manufacturing process of a tumour vaccine from a lysate from sources having cancer cells comprising the steps:
a) collecting tumour cells or tumour tissue from the sources, such as organs, dissected tumours, tissue bearing a living tumour or tumour cell lines,
b) transfer the collected tumour cells or tumour tissue of step a.) in a medium containing sodium chloride, potassium chloride, magnesium chloride, histidin/histidin hydrochloride, stabilizers, such as mannitol, calcium chloride, amino acids, such as tryptophane, 2-keto-glutaric acid or its physiologically acceptable salts, optionally physiologically acceptable auxiliary substances, and
c) preparation of the tumour vaccine.

## Description

The present invention pertains to a method for improving the manufacturing process of a tumour vaccine made from tumour cells, dissected living tumours, tissues bearing a tumour, or (tumour) cell lines and the use of a medium for transportation of these.

At present, tumour vaccine preparation typically starts with the isolation of tumour cells or tumour tissue from organs having developed cancer. These cells or tissues are then transferred into cell culture media.

Cell culture media have two major tasks:
1. they have to provide cells with the appropriate nutrients and oxygen. They also support cells in the production of distinct molecules like proteins by providing the respective precursors.
2. They have to remove, dilute or buffer metabolised by-products. To fulfil these requirements, cell culture media have to be isotonic and isomolar at physiological pH.

Thus, cell culture media are typically composed of compounds such as carbohydrates (glucose or derivatives), amino acids (precursors of proteins) and buffering systems such as phosphate salts. A large variety of such media is commercially available. It is the task of such media to generate conditions under which cells have a strong physiologic activity marked e.g. by high metabolic turnover, proliferation or motility

However, a need exists for media which are more suitable for the transport, storage and shipment of isolated living tumour cells, dissected living tumours, tissues bearing a living tumour, or (tumour) cell lines.

One object of the invention was to provide a method satisfying the existing need.

The object is accomplished by a method for improving the manufacturing process of a tumour vaccine including, but not limited to,the steps of:
a.) collecting tumour cells or tumour tissue from the sources, such as organs, dissected tumours, tissue bearing a living tumour or tumour cell lines,
b.)transfer the collected tumour cells or tumour tissue of step a.) in a medium containing sodium chloride, potassium chloride, magnesium chloride, histidin/histidin hydrochloride, stabilizers, such as mannitol, calcium chloride, amino acids, such as tryptophane, 2-keto-glutaric acid or its physiologically acceptable salts, optionally physiologically acceptable auxiliary substances.

Unexpectedly, media which are currently used for the transport and shipment of solid organs for transplantation are also suitable for the transport, storage and shipment of isolated tumour cells, dissected tumours, tissues bearing a living tumour, or (tumour) cell lines.

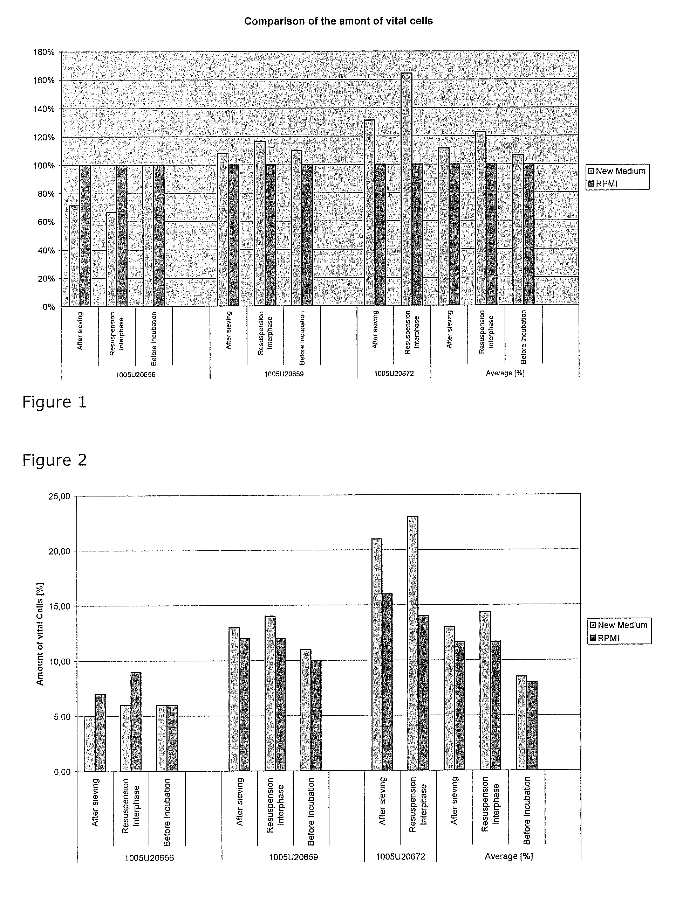

Figure 1 depicts the number of vital cells received relative to the standard medium used for tumour transport (RPMI).

Figure 2 depicts the respective absolute numbers.

As given above, standard cell culture media put cells in a condition to be physiological active. Physiologic activity is generally accompanied by the need for energy which is satisfied by metabolizing carbohydrates into acidic and therefore toxic metabolic by-products. According to the invention, media with a low amount of carbohydrates protect the cells from metabolic damage. The use of low amounts of carbohydrates additionally avoids oxidative bursts and the formation of respective toxic metabolites (radicals). Another way to protect the cells is to use higher concentrations of potassium ions in order to reduce activities via the membrane potential. Further electric membrane signalling can be reduced by applying higher concentrations of sodium salts.

In one embodiment of the invention, the tumour cells or tumour tissue are collected from solid tumours or non-solid tumours.

In particular, the tumour cells or tumour tissue are collected from kidneys, liver, lung, prostate, brain, gastrointestinal tract, respiratory tract, skin, head and neck, bone, bone marrow, pleura, peritoneum, secretory glands and/or mouth. The invention is further illustrated herein below by solid tumours from kidneys.

It is also possible to collect the tumour cells or tumour tissue from tumour cell lines which are available from depository institutions or may be individually cultured. The tumour vaccine may be derived from autologous allogenic or xenogenic sources.

As of yet, no media have been described to be exclusively suited for the storage, transport and shipment of isolated living tumour cells, dissected living tumours, tissues bearing a living tumour or (tumour) cell lines. The medium Custodiol^{®}, approved for the transport and shipment of solid organs for transplantation, is the only available drug for such a need.

Cells or Tissues which are e.g. incubated with stimulating agents like cytokines, subjected to genetic or other modifications or amplified by proliferation need to be vital. Using these media, a higher degree of vitality can be achieved during the manufacturing process. The variability of the process can be decreased, also. This allows a much better efficacy and standardization of any process requiring vital or at least non-apoptotic or necrotic cells in some steps.

It can not be excluded that minimal amounts of the shipment media are present even in the final product. In order to avoid any toxic effects by the product, only non toxic substances may be used for the composition of an acceptable media. Also substances of animal origin (i.e. fetal calf serum, enzymes etc.) have to be excluded due to a potential TSE-risk. Therefore, appropriate media allowing their incorporation in minimal amounts have to be used. Again, the commercially available medium Custodiol^{®} fulfils these requirements.

As an example of the invention, the tumour cells or tumour tissue are collected from kidneys. When the tumour bearing kidney is removed, the tumour is dissected by the surgeon with sterile equipment. Tumour samples are taken from the proliferation zone and placed in a medium bottle, filled with the Custodiol^{®} medium. The bottle is closed, sealed and shipped to the manufacturer of the tumour vaccine within a defined time frame (normally 24 h) for the production of the vaccine.

The molar composition of a preferred medium is as follows:

15.0 mmol sodium chloride, 9.0 mmol potassium chloride, 4.0 mmol magnesium chloride, 18.0 mmol histidine hydrochloride, 180.0 mmol histidine, 2.0 mmol tryptophane, 30.0 mmol mannitol, 0.015 mmol calcium chloride, 1.0 mmol potassium hydrogene-2-oxopentandioat in aqueous solution for injection.

The invention is further described in more detail by the following nonlimiting examples.

### Example 1

### Collection of tumour tissue

The tumour bearing kidney of the patient is removed during a surgical procedure. The kidney is than prepared in a sterile way to display the proliferation zone of the tumour. From the proliferation zone, tissue is being prepared and placed under sterile conditions in the tumour transport bottle containing the appropriate medium.

### Example 2

### Transportation of tumour tissue

Shipment of the medium to the clinic and of tumour tissue to manufacturer is carried out using 250 mL polystyrene bottles closed with caps manufactured from high density polyethylene according to USP 24 class VI.

The bottles for the transport of medium and tumour material are packaged into ColdSAFE Modules Plus containing cooling accumulators providing a suitable temperature for shipment.

### Example 3

### Preparation of the tumor vaccine

The tumour vaccine is prepared as described in EP-B-13050411 comparing patients material shipped in the new medium versus RMPI-medium.

Samples were taken after sieving, after resuspension of the interphase and before the incubation step with interferon-gamma. The amount of vital cells after sieving was 11.43%, after resuspension of the interphase 22.86% and before the incubation 6.25% higher compared to the preparation made from RPMI-shipped tumour.

## Claims

1. A method for improving the manufacturing process of a tumour vaccine from a lysate from sources having cancer cells comprising the steps:
a) collecting tumour cells or tumour tissue from the sources, such as organs, dissected tumours, tissue bearing a living tumour or tumour cell lines,
b) transfer the collected tumour cells or tumour tissue of step a.) in a medium containing sodium chloride, potassium chloride, magnesium chloride, histidin/histidin hydrochloride, stabilizers, such as mannitol, calcium chloride, amino acids, such as tryptophane, 2-keto-glutaric acid or its physiologically acceptable salts, optionally physiologically acceptable auxiliary substances, and
c) preparation of the tumour vaccine.

2. The method of claim 1, wherein tumour cells or tumour tissue are collected from solid tumours.

3. The method of claim 1, wherein tumour cells or tumour tissue are collected from non-solid tumours.

4. The method of any one of the claims 1 to 3, wherein tumour cells or tumour tissue are collected from kidneys, liver, lung, prostate, brain, gastrointestinal tract, respiratory tract, skin, head and neck, bone, bone marrow, pleura, peritoneum, secretory glands and/or mouth.

5. The method of claim 2, wherein tumour cells or tumour tissue are collected from solid tumours from kidneys.

6. The method of any one of the claims 1 to 5, wherein tumour cells or tumour tissue are collected from tumour cell lines.

7. The method of any one of the claims 1 to 6, wherein the tumour vaccine is autologous.

8. The method of any one of the claims 1 to 6, wherein the tumour vaccine is allogenic.

9. The method of any one of the claims 1 to 6, wherein the tumour vaccine is xenogenic.

10. The method according to any one of the claims 1 to 9, wherein the medium contains in 1000 ml solution the following ingredients: 15.0 mmol sodium chloride, 9.0 mmol potassium chloride, 4.0 mmol magnesium chloride, 18.0 mmol histidine hydrochloride, 180.0 mmol histidine, 2.0 mmol tryptophane, 30.0 mmol mannitol, 0.015 mmol calcium chloride, 1.0 mmol potassium hydrogene-2-oxopentandioat in aqueous solution for injection.

11. Use of a medium containing sodium chloride, potassium chloride, magnesium chloride, histidin/histidin hydrochloride, stabilizers, such as mannitol, calcium chloride, amino acids, such as tryptophane, 2-keto-glutaric acid or its physiologically acceptable salts, optionally physiologically acceptable auxiliary substances for the transportation isolated tumour cells or tumour tissue of an organ having a tumour.
